(19) Europäisches Patentamt
European Patent Office
Office européen des brevets

(11) Veröffentlichungsnummer: **0 150 441**
**B1**

(12) # EUROPÄISCHE PATENTSCHRIFT

(45) Veröffentlichungstag der Patentschrift: **18.03.87**

(51) Int. Cl.⁴: **C 07 C 121/50,** D 06 L 3/12

(21) Anmeldenummer: **84115777.9**

(22) Anmeldetag: **19.12.84**

(54) **1,4-Bis-(styryl)-benzole und deren Verwendung als optische Aufheller.**

(30) Priorität: **30.12.83 DE 3347576**

(43) Veröffentlichungstag der Anmeldung: **07.08.85 Patentblatt 85/32**

(45) Bekanntmachung des Hinweises auf die Patenterteilung: **18.03.87 Patentblatt 87/12**

(84) Benannte Vertragsstaaten: **CH DE FR GB IT LI**

(56) Entgegenhaltungen:
**EP-A-0 032 254**
**DE-A-3 119 992**
**FR-A-2 251 549**

**Ullmanns Encyklopädie der technischen Chemie, 4. Auflage, Band 17 (1979), Seiten 461-462**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Dorlars, Alfons. Dr., Mozartstrasse 32, D 5090 Leverkusen (DE)**
Erfinder: **Schellhammer, Karl- Wolfgang. Dr., Katharinental 26, D 5060 Bergisch- Gladbach 2 (DE)**
Erfinder: **Brinkwerth, Wolfgang. Dr., Am Hang 21, D 5090 Leverkusen (DE)**

**EP 0 150 441 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Gegenstand der Erfindung sind Verbindungen der Formel

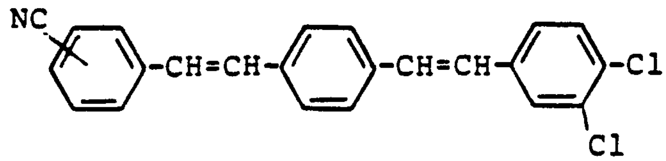

(I)

sowie deren Mischungen untereinander und mit strukturell ähnlichen oder anwendungstechnisch artverwandten anderen optischen Aufhellern.

Bevorzugte Aufheller sind solche der Formel I, worin die Nitrilgruppe in p- oder o-Stellung steht, wobei das letztgenannte Isomere besonders bevorzugt ist.

Von besonderem praktischen Interesse sind Mischungen von Bisstyrylbenzolen bestehend aus 50 - 70 % der Verbindung der Formel

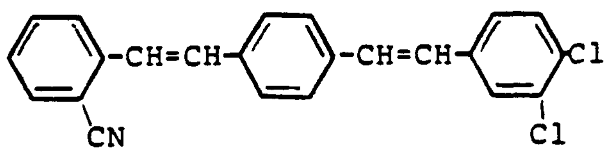

(IIa)

25 - 15 % der Verbindung der Formel

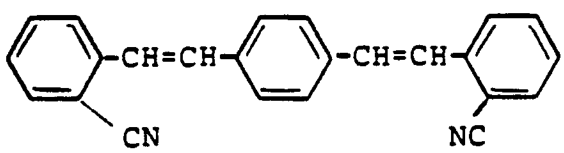

(IIb) und

25 - 15 % der Verbindung der Formel

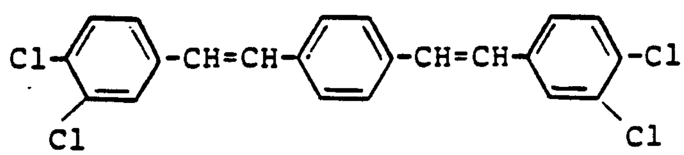

(IIc)

Die Verbindungen der Formeln IIb und IIc sind literaturbekannt (vgl. z.B. DAS 1 444 003, Tabelle).

Auch die Herstellung der Einzelverbindungen (I) und der Gemische II ist im Prinzip bekannt (vgl. DAS 1 112 072, DAS 1 108 219, EP-A 23 027, EP-A 30 917 und EP-A 32 254).

Vorteilhafterweise werden die einzelnen Individuen der Formel I nach dem im nachfolgenden Beispiel angegebenen Verfahren hergestellt. Darüber hinaus sind diese Verbindungen auch nach dem Verfahren gemäß EP-A 32 254 zugänglich, wobei Terephthaldehyd nacheinander mit den beiden entsprechenden Benzylphosphonsäureestern in einem geeigneten Lösungsmittel und in Gegenwart von Alkali kondensiert werden. Allerdings sind die dabei erhaltenen Produkte mit den jeweiligen symmetrischen Komponenten verunreinigt, deren Abtrennung Schwierigkeiten bereitet.

Die Gemische der Formeln IIa - IIc können grundsätzlich durch mechanisches Abmischen der Einzelkomponenten hergestellt werden.

Zweckmäßigerweise erfolgt jedoch die Herstellung dieser Mischungen durch einstufige Kondensation von Terephthalaldehyd mit der berechneten Menge eines Gemisches der entsprechenden Benzylphosphonsäureester - vorzugsweise in einem polaren aprotischen organischen Lösungsmittel und in Anwesenheit eines basischen Katalysators.

Die neuen Verbindungen der Formel I und insbesondere die sich davon ableitenden technisch besonders gut zugänglichen Gemische der Formel II eignen sich hervorragend zum optischen Aufhellen von Synthesefasermaterialien, insbesondere Polyesterfasern, nach üblichen Methoden, wobei sie sich durch ein hohes Weißmaximum auszeichnen. Hervorzuheben ist im übrigen, daß die neuen Aufheller bereits im unteren Temperaturbereich beim Auszieh- und Thermosolfärben (geringere Kantenmarkierung) gut entwickeln. In dieser Hinsicht sind die erfindungsgemäßen Verbindungen auch nächstvergleichbaren, aus EP-A 32 254, DE-A 31 19 992 und FR-A 2 251 549 bekannten Aufhellern überlegen.

Sowohl die Einzelkomponenten der Formel I, z. B. II a, als auch deren Mischungen mit II b und II c, wie sie in einstufiger Kondensation von Terephthalaldehyd mit den entsprechenden Benzylphosphonsäureestern anfallen, können ihrerseits in Mischungen beliebiger Zusammensetzung mit weiteren Polyester-Aufhellungsmitteln in Auszieh-, Thermosol- oder Einspinnverfahren angewendet werden. Das kann sich z. B. als zweckmäßig erweisen, um anwendungstechnischen Forderungen nach Nuance, Echtheiten, Wirtschaftlichkeit oder ähnlichem zu genügen.

**Beispiel 1**

1-[2-Cyanstyryl]-4-[3',4'-dichlorstyryl]-benzol

a) 2-Cyan-4'-methyl-stilben:

In vorgelegte

60 g (0,33 Mol) Natriummethanolatlösung 30 %ig tropft man bei 25 - 30° C unter Rühren eine Lösung von

30 g (0,25 Mol) 4-Methylbenzaldehyd und

64 g (0,25 Mol) 2-Cyan-benzylphosphonsäurediethyl-ester in

100 ml Dimethylformamid. Man rührt 8 Stunden bei Raumtemperatur und gibt dann

200 ml Wasser hinzu, worauf das beigefarbene Stilbenderivat ausfällt. Es wird abgesaugt, mit Wasser gewaschen und getrocknet.

Ausbeute: 40 g beigefarbenes Pulver.

Fp: 60 - 61° C (Lit.)

b) 2-Cyan-4'-formyl-stilben:

37 g (0,17 Mol) des erhaltenen rohen 2-Cyan-4-methylstilbens löst man in

400 ml Tetrachlorkohlenstoff, fügt

30 g (0,17 Mol) N-Bromsuccinimid und

1 g Azo-isobutyronitril hinzu und kocht das Gemisch unter Rückflußkühlung, bis das NBS verbraucht ist (NBS ist schwerer, Succinimid leichter als $CCl_4$). Nach Abkühlen auf 25° C filtriert man vom Succinimid ab, wäscht es mit

50 ml Tetrachlorkohlenstoff aus und gibt zum Filtrat eine Lösung von

40 g (0,28 Mol) Hexamethylentetramin in

300 ml Chloroform. Man kocht das Gemisch 1 Stunde unter Rückflußkühlung, stellt den Ansatz auf 15° C und saugt das auskristallisierte Quartärsalz ab. Darauf suspendiert man es in

800 ml 50 %iger Essigsäure, gibt weitere

15 g (0,1 Mol) Hexamethylentetramin hinzu und kocht noch 2 Stunden am Rückflußkühler.

Dann werden

400 ml Wasser zugegeben und der nach Abkühlen auf 15° C auskristallisierte rohe 2-Cyano-stilben-4'-aldehyd wird aus Ethanol/Kohle umkristallisiert.

Ausbeute: 14,8 g (37 % d. Th.)

Fp: 151 - 152° C (Ligroin).

c) Cyanstyryl-dichlorstyryl-benzol:

14,8 g (0,06 Mol) 2'-Cyano-4-formyl-stilben werden zusammen mit

20 g (0,067 Mol) 3,4-Dichlorbenzyl-phosphonsäure-diethylester in

50 ml Dimethylformamid gelöst. Dazu tropfen unter Rühren bei 25° C

14 g (0,08 Mol) Natriummethanolatlösung 30 %ig; nach 8-stündigem Rühren bei 25 - 30° C werden zu der selben Suspension

50 ml Methanol zugegeben. Man saugt den Niederschlag ab, wäscht mit

50 ml Methanol und 50 ml heißem Wasser nach und trocknet im Vakuum: 20 g Rohprodukt. Zur Reinigung kristallisiert man nach Klärung mit Bleicherde aus Chlorbenzol um.

F. 183 - 185° C.

Ausbeute: 17,4 g grünstichiggelbes Kristallpulver (77,1 % d.Th.)

Aus dem Filtrat gewinnt man noch 2,5 g weniger reines Produkt.

**Beispiel 2**

196 g (1,0 Mol) 3,4-Dichlor-benzylchlorid werden bei 40° C unter Rühren mit

152 g (1,0 Mol) 2-Cyan-benzylchlorid (99,5 %ig) vermischt. Die erhaltene Schmelze läßt man unter Rühren in

349 g (2,10 Mol) Triethylphosphit, das auf 140° C erhitzt wurde, eintropfen. Gleichzeitig leitet man Stickstoff über die Oberfläche der Reaktionsmischung. Man rührt noch 5 Stunden bei 140 - 150° C in schwachem Stickstoffstrom und unter Rückflußkühlung. Das Benzylphosphonat-Gemisch läßt man dann auf Raumtemperatur abkühlen und entspannt mit Stickstoff. Zum Rückstand läßt man

900 ml Dimethylformamid zulaufen und trägt

123 g (0,917 Mol) Terephthalaldehyd (100 %ig) ein. Zu dieser Lösung tropft man bei 25 - 30° C unter

Stickstoffatmosphäre und Rühren
391 g (2,17 Mol) Natriummethanolatlösung (30 Gew.-%ig).
Nach kurzer Zeit beginnt die Abscheidung der gelben Distyrylbenzole. Man rührt über Nacht bei 25 - 30°C. Danach läßt man
1080 ml Methanol in die Suspension einlaufen. Anschließend kühlt man auf 25°C, saugt das ausgeschiedene Produkt ab, wäscht mit Methanol und dann mit heißem Wasser. Nach dem Trocknen erhält man 290 g Bisstyrylbenzolgemisch, das zwischen 150 und 163°C schmilzt (= 83,3 % der Theorie, bezogen auf Terephthalaldehyd, bzw. 76,5 % der Theorie, bezogen auf eingesetzte Benzylchloride).

**Beispiel 3**

Verfährt man wie in Beispiel 1, setzt jedoch in a) 64 g 4-Cyan-benzylphosphonsäureester ein, so erhält man am Schluß das 1-[4-Cyanstyryl]-4-[3',4'-dichlorstyryl]benzol, das weniger günstig ist als das 2-Cyan-Isomere.

**Beispiel 4**

Garn aus Polyesterfasern wird in einem Färbeautoklaven im Flottenverhältnis von 1:25 mit einem Bade folgender Zusammensetzung behandelt: 0,1 % (des Garngewichtes) des im Beispiel 2 beschriebenen Bisstyrylbenzolgemisches in feindispergierter Form, 1 g eines ethoxylierten Stearylalkohols und 1 l enthärtetes Wasser. Man erhitzt die Flotte allmählich auf 120°, hält diese Temperatur 30 Minuten, kühlt dann ab, spült und trocknet.
Man erhält ein stark aufgehelltes Garn von leicht rotstichiger Nuance und gutem Echtheitsniveau.

**Beispiel 5**

Ein Gewebe aus Polyethylenglykol-terephthalat wird mit einer wäßrigen Suspension, die im Liter 1 g des in Beispiel 1 beschriebenen Distyrylbenzolderivates, 2 g handelsübliches Dispergiermittel und 1 g handelsübliches Netzmittel enthält, bis zu einer Flottenaufnahme von 80 % (bezogen auf das Warengewicht) imprägniert und einer Thermosol-Behandlung unterzogen. Das Gewebe zeigt gegenüber unbehandelter Ware eine klare, starke, leicht rotstichige Aufhellung von guter Licht-, Naß- und Chloritechtheit.

**Beispiele 6 - 15**

Ein Gewebe aus Polyester-Fasern wird mit einer Weißtöner-Suspension, die im Liter eine Mischung der in der Tabelle angegebenen Mengen der erfindungsgemäßen mit bekannten Verbindungen, ferner 2 g eines handelsüblichen Dispergiermittels und 1 g eines handelsüblichen Netzmittels enthält, bis zu einer Flottenaufnahme von 80 % imprägniert und anschließend thermosoliert. Man erhält starke Aufhellungen mit gutem Echtheitsniveau.

| Beispiel | Erfindungsgemäßer Aufheller | Bekannter Aufheller |
|---|---|---|
| 6 | 0,3 g Beispiel 2 | 1,4 g 3-Phenyl-7-[4-phenyl-5-methyl-v-triazolyl-(2)]cumarin |
| 7 | 0,3 g Beispiel 2 | 0,7 g 3-[4-Chlorpyrazolyl-(1)]-7-[4-phenyl-5-methyl-v-triazolyl-(2)]-cumarin |
| 8 | 0,5 g Beispiel 1 | 0,4 g 1.4-Bis-[2-cyanstyryl]-benzol |
| 9 | 0,5 g Beispiel 2 | 0,4 g 1-[4-Cyanstyryl]-4-[2-cyanstyryl]-benzol |
| 10 | 0,7 g Beispiel 2 | 0,45 g 3-[4.6-Dimethoxy-1.3.5-triazinyl-(2)]-pyren |
| 11 | 0,3 g Beispiel 2 | 2,6 g 1.2-Bis-[5-methyl-benzoxazolyl-(2)]-ethen |
| 12 | 0,5 g Beispiel 2 | 1,6 g 2.5-Bis-[benzoxazolyl-(2)]-thiophen |
| 13 | 0,5 g Beispiel 2 | 0,5 g 1.4-Bis-[benzoxazolyl-(2)]-naphthalin |
| 14 | 0,4 g Beispiel 2 | 4 g 4-Methoxy-1.8-naphthalsäure-N-methylimid |
| 15 | 0,8 g Beispiel 2 | 0,24 g 4-[Benzoxazolyl-(2)]-4'-[3-methyl-1.2.4-oxdiazolyl-(5)]-stilben |

**Patentansprüche**

1. Verbindungen der Formel

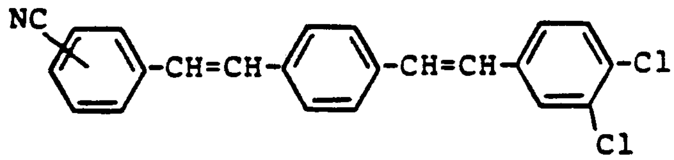

(I)

2. Verbindung gemäß der in Anspruch 1 angegebenen Formel, worin sich die Cyangruppe in o-Stellung zur Ethenylenbrücke befindet.

3. Verbindungen der Formel 1 im Gemisch miteinander oder mit strukturell oder anwendungstechnisch ähnlichen optischen Aufhellern.

4. Mischungen von Bisstyrylbenzolen bestehend aus 50 - 70 % der Verbindung der Formel

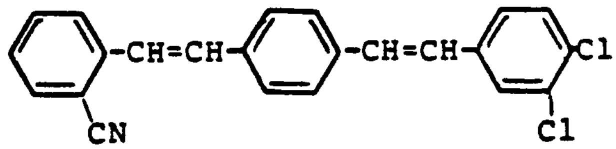

(IIa)

25 - 15 % der Verbindung der Formel

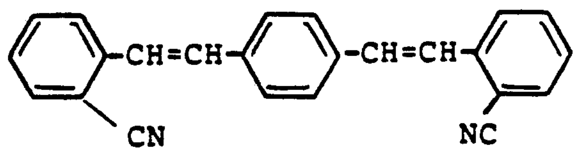

(IIb) und

und 25 - 15 % der Verbindung der Formel

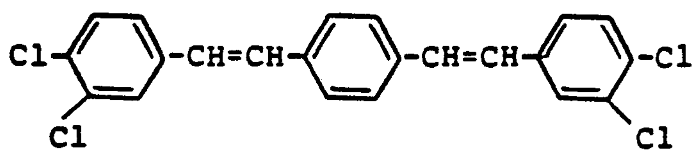

(IIc)

5. Verwendung der Verbindungen bzw. Mischungen der Ansprüche 1 - 4 zum optischen Aufhellen von Polyesterfasern.

**Claims**

1. Compounds of the formula

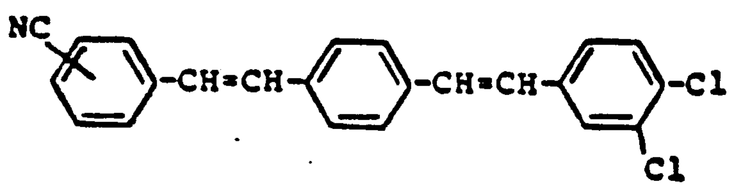

(I)

2. Compound according to the formula given in Claim 1, wherein the cyano group is in the o-position with respect to the ethylene bridge.

3. Compounds of the formula I as a mixture with one another or with optical brighteners which are structurally similar or are used similarly.

4. Mixtures of bisstyrylbenzenes consisting of 50-70% of the compound of the formula

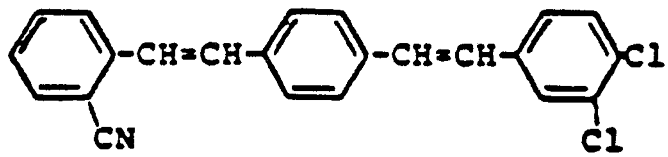

(IIa)

25-15% of the compound of the formula

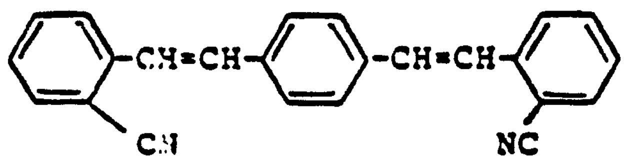

(IIb) and

and 25-15% of the compound of the formula

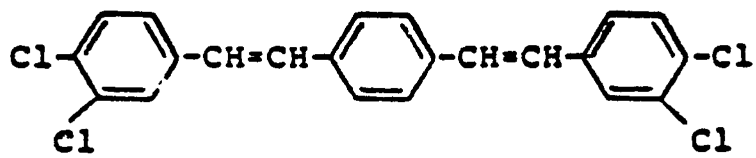

(IIc)

5. Use of the compounds or mixtures of Claims 1-4 for the optical brightening of polyester fibres.

**Revendications**

1. Composés de formule

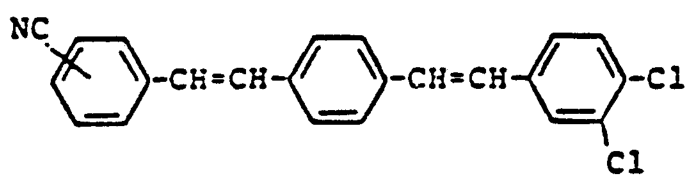

(I)

2. Composé répondant à la formule donnée dans la revendication 1, dans laquelle le groupe cyano est en position o par rapport au pont éthénylène.

3. Composés de formule I en mélange entre eux ou avec les azureurs optiques voisins par leur structure ou par leurs applications techniques.

4. Mélanges de bis-styryl-benzènes consistant en:

50 à 70 % du composé de formule

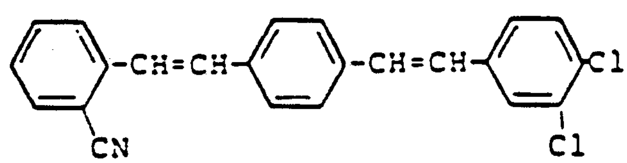

(IIa)

25 à 15 % du composé de formule

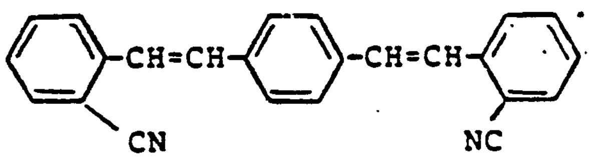

(IIb) et

et 25 à 15 % du composé de formule

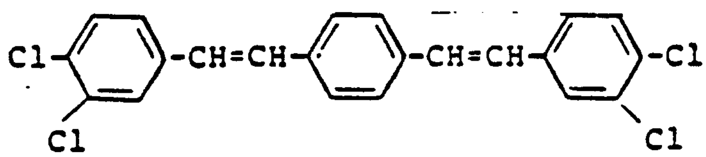

(IIc)

5. Utilisation des composés ou mélanges selon les revendications 1 à 4 pour l'azurage optique de fibres de polyesters.